# EUROPEAN PATENT SPECIFICATION

(11) **EP 0 447 569 B1**
(45) Date of publication and mention of the grant of the patent: **05.10.1994**
(21) Application number: 90104692.0
(22) Date of filing: 13.03.1990
(51) Int. Cl.: A61K 33/26

(54) **Product for the prophylaxis, diagnosis and therapy of rheumatic, autoimmune, skin and connective tissue diseases of an unknown aetiology, and methods of its manufacture and use**
Mittel zur Vorbeugung, Diagnose und Therapie von rheumatischen, autoimmunen, Haut- und Bindegewebekrankheiten von unbekannter Aetiologie und Verfahren zu seiner Herstellung und Verwendung
Produit pour la prophylaxie, le diagnostic et la thérapie des maladies rhumatismales, autoimmunes, de la peau et du tissu conjonctif d'étiologie inconnue, ainsi que sa méthode de fabrication et d'utilisation

(43) Date of publication of application: 25.09.1991
(73) Proprietor: Riha, Jara Ores, London SW7 3DQ (GB)
(72) Inventor: Riha, Jara Ores, London SW7 3DQ (GB)

(56) References cited:
- EP-A- 0 025 721
- EP-A- 0 246 734
- EMBASE ABSTRACT NO: 82 227 356 SCHIANO A. et al.: "Routine biologic investigations in rheumatoid arthritis (200 cases)

## Description

Product for the prophylaxis,diagnosis and therapy of rheumatic,autoimmune,skin and connective tissue diseases of an unknown aetiology, and methods of its manufacture and use.

This invention concerns the prophylaxis, diagnosis and therapeutic treatment of rheumatic, autoimmune, skin and connective tissue diseases of an unknown aetiology, and so it belongs to the realms of medical and veterinary rheumatology, immunology, immunopathology and dermatology.

The groups of diseases to which the present invention relates comprise a multitude of maladies, most of them being of an unknown or uncertain aetiology, many being chronic and intractable. Disadvantages and inadequacies existing in the state of art of prophylaxis, diagnosis and therapy of most of these maladies may be summed up as follows.
(a) It is not only that the causative agent (primary stimulus, persistent antigen) of most of these maladies has not been isolated nor identified, but hitherto it has not even been determined with certainty what type of microorganism, if any at all, is implicated as the aetiological agent. Numerous theories and hypotheses about the aetiology of rheumatic, autoimmune, skin and connective tissue diseases have held sway for varying periods of time. This situation may be exemplified by the following quotation: "... rheumatoid arthritis remains a mystery within a mystery and still without a master ... " (Bywaters E.G.L., The recent, spectacular history of rheumatoid arthritis and rheumatology from about 1928 onwards as seen through English eyes - and a pair of spectacles. In Utsinger P.D. et al. (eds), Rheumatoid Arthritis, J.B.Lippincott Company, Philadelphia, 1985, P.5).
(b) Furthermore, hitherto it has not even been known in which tissue or organ of the body the causative agent resides. Generally, the search for it has been focused on the tissues and/or organs showing clinical symptoms of the malady, e.g., on the joint in rheumatoid arthritis, while the apparently healthy parts of the body were passed by.
(c) One of the consequences of an unknown aetiology of most rheumatic, autoimmune, skin and connective tissue diseases has been the uncertainty about their contagiousness, whether or not they can be transmitted and by which routes and portals of entry, and how long the incubation or latent period can last.
(d) Hitherto, no effective prophylactic measures against most of these maladies have been available.
(e) Hitherto, it has been difficult, or impossible, to diagnose most rheumatic, autoimmune, skin or connective tissue diseases at their very beginning or during their latent period twenty or thirty years before the onset of clinical symptoms.
(f) Hitherto, no remedy has been available capable of eradicating the causative agent (primary stimulus, persistent antigen) of these maladies from the body and thus arresting the progress of the malady and curing the sufferer.
   Hence,diverse medical treatments have necessarily consisted mostly of a pain therapy and palliatives directed towards alleviation of symptoms, for example, anti-inflammatory drugs in rheumatoid arthritis, or restorative surgery using prostheses to replace destroyed joints.
   In some skin diseases, the treatments have been aimed at what may be a superinfection of lesions with dermatophytes, as in tinea, or with bacterial flora, as in acne. Such treatments usually do not eliminate the underlying cause of the clinical symptoms, that is the latency-reactivation sites embedded in the skin and the subcutaneous tissue very close to where a chronic fissuring or pustules occur.
   Failing an unpredictable remission, many of the rheumatic, autoimmune, skin and connective tissue diseases have been considered practically incurable and as afflictions to be inevitably endured in old age by a portion of the population.
(g) Hitherto, it has not been recognized in theory, nor in practice, that most of the rheumatic, autoimmune, skin and connective tissue diseases may be linked by a common causative agent, a one which is capable of bringing about different clinical symptoms in different subjects depending on the genetic characteristics of their immune system and its history. For example, rheumatoid arthritis, acne vulgaris and tinea pedis have been regarded as aetiologically unrelated and unconnected diseases.
(h) Some of the systemic drug therapies can have adverse side effects and induce complications. For example, the use of anti-inflammatory drugs may lead to ocular complications: in case of corticosteroids, glaucoma may occur; in case of antimalarial agents, even blindness (Burke M.J., Ocular manifestations. In Utsinger P.D. et al. (eds), Rheumatoid Arthritis, J.B. Lippincott Company, Philadelphia, 1985, p.363).

The present invention aims to put an end to the elusiveness of the causative agent (primary stimulus, persistent antigen) that has been at the core of the difficulties experienced in the state of art of prophylaxis, diagnosis and therapy of rheumatic, autoimmune, skin and connective tissue diseases. For this purpose, in a practical manner, it provides a product, as claimed in Claim 1, which consists of a salt, or salts, of iron (Fe) and a lipid, or lipids, that had all been heated together in a stirred vessel to the boiling point of the composition at the atmospheric pressure in the presence of water which subsequently may have been completely or partly removed from the product by distilling it off.

A salt of iron usable in accordance with the invention needs to have a physiologically harmless anion. A highly water soluble ferric salt is preferred.

An usable lipid may be selected from physiologically harmless fats, oils, waxes, phospholipids, glycolipids, terpenes, steroids, carboxylic acids and their esters, mono- and polyhydroxy alcohols and their ethers, polymers and copolymers of alkene oxides and their ethers. Preferred among these are nondrying edible oils.

Said product is distinguished by its property of eliciting and curing lesions at asymptomatic latency-reactivation sites in which resides the causative agent of rheumatic, autoimmune, skin and connective tissue diseases while it evokes no such reaction in the rest of the skin.

For this purpose, said product may be used as it is and applied directly to the human or animal skin. It may also be incorporated as active ingredient into ointments, creams, pastes, salves, gels, lotions, rinses, emulsions, aerosols sprays, bath additives, washes, dispersions and other compositions intended for a topical application to the human or animal skin. In addition, said product may be contained in devices which are to be applied to the human or animal skin and which include pads, dressings, compresses, poultices,napkins.

Whichever way said product is applied to the skin, it has the effect of revealing by lesions it induces where the latency-reactivation sites are, as well as curing the lesions and blocking the establishment of the causative agent in the skin,as claimed.The latency-reactivation sites are in many cases asymptomatic, and so the skin may appear and feel quite normal prior to a topical treatment with said product notwithstanding it may harbour hundreds of these sites.

The latency-reactivation sites,in which the causative agent can persist for years and decades of the host's life, are disseminated segments of the skin and the subcutaneous tissue frequently of a conical or wedge-like shape with the thin end pointing into the tissue and the apex reaching an estimated maximal depth of about 15mm. The base of the cone lies at the skin's surface and it may be up to about 25mm in diameter, but of an estimated median diameter of about 7mm.

Frequently, the latency-reactivation sites are found in intertriginous regions of the skin (commonly, one site being exactly opposite and in touch with another one which faces it), in wrinkles, furrows and skin folds, in the nail grooves and walls, in the hairy skin, the beard and the scalp.

In particular, the following skin regions are likely to harbour latency-reactivation sites: the skin of the toeclefts, the dorsum of the toes, the toenail grooves and walls; the skin of the dorsum of the feet, the shanks (especially around the ankles) and the knees; the skin of the perineum, the groin, the genitalia (except the glans penis) and the perianal skin; the skin of the dorsum of the hands and the wrist, the nail grooves and walls; the skin of the back of the neck, the entire face (especially, the nose and the nostrils) and the scalp. In addition, pimples, pustules, papules, macules, comedos, lentigines (freckles or "sunspots"), peeling, scurf, fissuring, itching, urticaria or prurigo, no matter how slight or insignificant these symptoms appear, may signal the existence of latency-reactivation sites in the vicinal skin.

A characteristic feature of the distribution of latency-reactivation sites over the skin of the human body is a pattern of an approximate bilateral symmetry. An important and consequential attribute of latency-reactivation sites is that they are embedded in the skin and the subcutaneous tissue, in some cases, overlain by layers of thickened skin or enclosed in callosities. Furthermore, they have a conical root extending deep into the subcutaneous tissue, which is the part of the latency-reactivation site that can resist the eradication by said product for many months since it becomes only slowly exposed at the skin's surface. The mechanisms operating in the growth of the latency-reactivation site and the reaction of surrounding tissue to it seem to converge into "burying" the site.

The necrosis of the latency-reactivation sites brought about by a topical dermal application of said product proceeds from the surface of the skin into the subcutaneous tissue.

It is believed that the portals of entry of the causative agent are the hair follicles and openings in the skin due to mechanical, chemical or enzymic injuries, and that after establishing itself in the skin, other cells in the vicinity are infected and the latency-reactivation site grows sending a root deeper into the tissue possibly to connect to a supply of nutrients from a blood vessel.

### Innovative solutions offered by the invention

The novelty of the invention lies in combining a salt, or salts, of iron (Fe) with a lipid, or lipids, in applying the product topically with the aim at eliciting a reaction in the skin of susceptible subjects in associating the skin reaction with certain maladies (thus suggesting for the first time that they may have a common factor), in recognizing the skin reaction as an indicator of these maladies in their latent stage and in making use of it for arresting their further development.

Hitherto, the only known topical application uses for iron salts were as astringents or styptics in a purely aqueous solution (see Martindale, The Extra Pharmacopoeia, 28th edition, 1982, pp. 872, 873 and 875).
(a) The present invention makes it feasible and practical to demonstrate that the causative agent of rheumatic, autoimmune, skin and connective tissue diseases actually exists as it provides a product which in a topical dermal application provokes a reaction of the infected or transformed cells.
(b) The invention makes it possible to pinpoint the sites in the skin where the causative agent asymptomatically resides. This opens a new and seminal direction for its eventual isolation, identification and cultivation in vitro, and for a more complete understanding of the aetiology and pathogenesis of rheumatic, autoimmune, skin and connective tissue diseases.
(c) The existence, the character and the distribution of the latency-reactivation sites, as they are revealed in the human skin by a topical dermal application of said product, strongly suggest that there is an infectious factor in the pathogenesis of most rheumatic, autoimmune, skin and connective tissue diseases with a latent period lasting up to several decades.
(d) Said product when applied to the skin as claimed makes for the first time feasible an effective prophylaxis of rheumatic, autoimmune, skin and connective tissue diseases by blocking the establishment in the skin of their, most probably ubiquitous, causative agent, its growth into the latency-reactivation sites and its spread by autoinfection to most parts of the integument.These processes may progress, waxing and waning, for decades with the effect of increasing the quantity of antigen existing in the body and the magnitude of immune response to it , often accompanied by chronic fatigue.
(e) During the latent period of many of these maladies, while their causative agent is colonizing the skin and the latency-reactivation sites are tiny and few, the clinical symptoms, which later are to overwhelm the patient, may be absent as, for example, in rheumatoid arthritis.
   The present invention provides a product for the diagnosis of rheumatic, autoimmune, skin and connective tissue diseases in their very early phase as well as for arresting their progress to a clinical stage. A periodical treatment of the skin with said product as claimed detects and eradicates in the initial stage of their growth the latency-reactivation sites which may not betray their presence in any apparent way or only by trivial pimples or fissuring in the skin (now, however, revealed by this invention as being of importance).
   Such diagnosis-cum-eradication of the latency-reactivation sites from the skin may have to be repeated at intervals throughout the lifetime of a person, especially if a genetic predisposition and living conditions favour the colonization of his/her skin by the causative agent.
(f) The therapy of rheumatic, autoimmune, skin and connective tissue diseases now being made possible by the present invention as claimed is radically different from the hitherto practised therapies insofar as they have been unable to target the aetiological agent which is now no longer inaccessible seeing that said product reaches it as it survives incognito in the skin and the subcutaneous tissue of the patient.
   This invention provides a product for eliminating from the body the causative agent of maladies for which hitherto has been no cure.
   From the sufferer's point of view, the fundamental difference is that managing a malady or treating its symptoms, instead of its cause, does not arrest its progress. From the medical science's point of view, the invention provides a new product which in an innovative step reveals that the primary stimulus or the persistent antigen is to be found in the skin - and not in those other organs which are showing clinical symptoms, such as, for example, the rheumatic joint.
(g) The present invention as claimed makes it feasible for the first time to demonstrate that many rheumatic, autoimmune, skin and connective tissue diseases have the same causative agent (primary stimulus, persistent antigen). Consequently, one of the innovative effects of the invention is that even mild skin ailments can no longer be regarded as having only a mere cosmetic or aesthetic importance.
(h) Considering the physiological properties of the ingredients from which said product is manufactured as claimed, it is extremely unlikely that it could cause any harmful side effects. Furthermore, the invention does not comprise a systemic administration of said product: a local concentration of therapeutically active substances, sufficiently high for eliciting a reaction in infected or transformed cells of the latency-reactivation sites, is achieved through a topical dermal application. Nothing was observed which would suggest that the rest of the skin, apart from the latency-reactivation sites, reacts to the application of said product to it.

### Advantageous effects of the invention

In addition to the innovative solutions which the invention offers in the field of the prophylaxis, diagnosis and therapy of rheumatic, autoimmune, skin and connective tissue diseases, other beneficial effects, such as the following ones, are expected to flow from its widespread use and industrial exploitation.
(a) The invention provides a product which is inexpensive to manufacture. This is a feature that should be of a particular benefit to the countries with scant resources enabling them to reduce their expenditure on imported medicines.
(b) In view of the enormous economic costs which the rheumatic, autoimmune, skin and connective tissue diseases inflict upon the humankind, if by using the present invention their prevalence was reduced even by a mere 1%, it would amount to a saving of hundreds of million U.S.dollars annually. For example, in the U.S.A. in 1979, the direct and indirect economic costs of rheumatoid arthritis alone were estimated at $US4315 millions (Straszheim M., Economic costs. In Utsinger P.D. et al. (eds), Rheumatoid Arthritis, J.B.Lippincott Company, Philadelphia, 1985, pp. 854-855).
(c) As the invention offers a product and a method of its application useful in the study of epidemiology and pathogenesis of maladies transmitted from the skin to the skin, it may contribute to the elucidation of the epidemiology and latency of Herpesviridae , HIV-1 and HIV-2.
(d) The invention by providing a product and a method of its use in detecting and eradicating slow-growing, often asymptomatic, latency-reactivation sites, may open a new line of investigation into oncogenes, latency and carcinomas.
(e) One of the effects of the invention may be to draw attention to the microbiology of the skin which, although it is an easily and directly accessible organ, might have been rather overlooked.
   There are indications that the microorganisms inhabiting the skin, especially around the body orifices, have a greater influence upon their host's health than has hitherto been realized. For example, the incidence and severity of respiratory ailments may be affected by the latency-reactivation sites and microbial population found around the lips and the nostrils.
   It is very likely that the application of said product, in addition to eradicating the latency-reactivation sites, also alters the composition and density of the skin's microbial population with the result that it could improve the state of the patient's health in a number of yet unexpected ways.
(f) The invention provides a prophylactic, diagnostic, therapeutic and research tool for veterinary medicine.

The following examples describe the invention in further details and illustrate its practice but they are not limitative of the scope of the invention nor of the ambit of the claims.

### Example 1

Manufacture of the product as claimed in Claim 1.

In a stirred vessel, fitted with a thermometer, condenser and a heating jacket, 150 grams of anhydrous ferric chloride (FeCl₃) are dissolved in 4 litres of water and 1 litre of sesame oil is added to the solution. The charge is heated under the atmospheric pressure to its boiling point. The heat input is then adjusted to maintain a steady rate of distillation of about 1 litre of distillate per hour. The distillation is continued until the water content of the charge in the vessel drops below 5% w/w. The distillate is discarded. The residue in the vessel is the product as claimed in Claim 1.

### Example 2

Preparation of a composition as claimed in Claim 2.

40 parts by weight of the product defined in Claim 1 are blended with 60 parts by weight of a pharmaceutically acceptable ointment base, such as Simple Ointment, B.P.

### Example 3

Manufacture of a therapeutic device as claimed in Claim 3.

A pad of cotton cloth, cut into a required size and provided with a strip of adhesive bandage, is impregnated with the product defined in Claim 1 or a composition according to Claim 2 or a prophylactic or diagnostic preparation and/or a medicine according to Claim 4 and packed in a disposable impermeable sleeve.

### Example 4

The use of the product defined in Claim 1 and of compositions and devices defined in Claim 2 to 4 which contain said product.

The invention to be effective requires that it is applied to the subject's skin. This may be carried out by using said product as it is, or a composition in which said product is an ingredient, or a device containing it and affixed to the skin so that said product is allowed to come into contact with the skin. These methods are not mutually exclusive and they may all be employed simultaneously.

The choice is made according to what is a most convenient and practical method, having regard to the configuration of the skin region to be treated and the lifestyle of the subject. For example, generally, it would not be practical to apply pads onto the scalp or the skin of the face where it is more convenient to use said product as it is or a cream in which said product is an ingredient. However, a pad or compress impregnated with said product and affixed to the skin by an adhesive bandage would be a good way of applying the invention to an indolent ulcer on the leg. From the point of view of lifestyle, pads and compresses may suit a person confined to bed or in a sedentary occupation, but not someone who is physically very active.

Regardless of the way said product is applied onto the skin, it is best to renew its application at least once a day after having washed the treated area of the skin with warm soapy water and debrided any lesions that may exist there.

An asymptomatic latency-reactivation site reacts to a topical dermal application of said product by becoming apparent initially as a prurient or urticant spot, pimple, pustule or papule which, in case of a larger site, usually develops into a, generally painless, ulcer. If a latency-reactivation site is very large or if there is a cluster of them, the surrounding tissue may swell for a day or two when a lesion is about to appear or has just appeared.

The resistance of asymptomatic latency-reactivation sites to the treatment with said product vary widely: in some sites, a reaction is brought forth in a few days, in others, it takes several weeks.

Once an ulcer is formed and debrided daily, it means that the objective of the present invention is being attained as the causative agent (primary stimulus, persistent antigen) of rheumatic, autoimmune, skin and connective tissue diseases is being removed from the skin with the necrosed tissue of latency-reactivation site. The application of said product, or a composition or device containing it, to the lesion is continued until it heals, which may take several weeks or months. Often, after it had healed, a pale scar remains where previously was a latency-reactivation site. It resembles one caused by smallpox vaccination and it does not seem to tan.

The use of the present invention is adapted to the state of health of the subject. However, in most cases it is best to treat one skin region after another, and not to apply the invention all over the entire integument simultaneously. The reason for this caution is to avoid, as far as possible, a syndrome of extreme fatigue, depression, loss of appetite and a sore throat which may precede or accompany the onset of extensive skin ulceration resulting from a simultaneous reaction of many latency-reactivation sites to said product.

For each of the main categories of subjects, one may proceed as follows in applying said product or composition or a device which contains said product.
(a) When the invention is used for prophylactic or diagnostic purposes on a subject with no clinical symptoms, it is most fruitful to begin by treating, one by one, the regions which are the most prone to the colonization by the causative agent of rheumatic, autoimmune, skin and connective tissue diseases. The density of lesions elicited in one or two of these areas of the skin is a good indication of the extent of overall colonization.
   The invention is consecutively applied to the above-mentioned skin regions where the latency-reactivation sites are to be found for about two weeks to each one, and lastly, the rest of the skin is treated. If no lesions are elicited, it may be provisionally interpreted that the area is free of the latency-reactivation sites. If many lesions appear in a skin region undergoing the treatment, it is best to wait before beginning to apply the invention to another part of the skin until the lesions that already erupted show signs of healing. Otherwise, one runs a risk of bringing forth many new lesions simultaneously, seeing that,when treating an asymptomatic skin region, one does not know prior to applying the invention to it whether it will elicit clusters of many lesions or just a few lesions or none at all.
   To effect the eradication of the latency-reactivation sites it is necessary to continue the application of the invention to the skin region under the treatment as long as any new lesions are being brought forth and/or the already elicited ones are not healed. As it may take months for some larger ulcers to heal, the treatment of the skin with the invention is pursued simultaneously in several skin regions in which lesions are healing and/or a few new ones are still appearing.
   This process of a progressive prophylaxis-cum-diagnosis and eradication of existing latency-reactivation sites is completed when the entire integument has been so tested. If in so doing,lesions have been elicited, then their number and size indicate the extent to which the subject's skin has been colonized by the causative agent of rheumatic, autoimmune, skin and connective tissue diseases.
   The diagnosis of asymptomatic latency-reactivation sites (by eliciting lesions at the sites) is a diagnosis of rheumatic, autoimmune, skin and connective tissue diseases in their latent phase. The interpretation of the findings is that the greater the number of lesions and their size (i.e., the greater the volume of infected or transformed cells), the higher is the probability that the subject will soon begin to present clinical symptoms of a rheumatic, autoimmune, skin or connective tissue disease, unless some factor intervenes,slowing or arresting the growth and spread of the sites or causing them to regress.
   In the latent phase of these diseases, the subject may for years carry a burden of latency-reactivation sites in her/his skin. Since they are often asymptomatic and, in any case, hitherto not known to exist, and since their clinical symptoms as, for example, acneic pustules, have hitherto been related to some other cause or remained unexplained, the subject has hitherto not been aware of the burden she/he carries.
   But, in many cases, the existence and proliferation of the latency-reactivation sites in the latent phase of rheumatic, autoimmune, skin and connective tissue diseases, is manifested by a recurrent or chronic fatigue and depression. Thus, even a partial eradication of the sites is likely to enhance the general well-being of the subject. A further development of these maladies is prevented if their causative agent (primary stimulus, persistent antigen) is removed from the skin by eradicating all, or practically all, latency-reactivation sites from it.
(b) In cases of subjects suffering from a connective tissue (collagen, mesenchymal) disease, such as systemic lupus erythematosus in which antigen-antibody complexes injure blood vessels, or from vasculitis, or from sarcoidosis in which immunoglobulins are deposited in various organs, or from amyloidosis with hypergammaglobulinaemia and localized accumulations of amyloid, the same procedure for the use of the invention may be followed as when it is used for prophylaxis/diagnosis of latency-reactivation sites in the skin of subjects with no clinical symptoms.
(c) In cases of subjects suffering from rheumatoid arthritis, osteoarthrosis or other rheumatic diseases, it may be best to apply the invention first to the skin over painful parts of the body, such as the joint, before proceeding to treat apparently normal skin regions as in cases of persons with no clinical symptoms. The reason for this is a probable nexus between an inflammed joint and the proximity of asymptomatic latency-reactivation sites from where the causative agent (primary stimulus, persistent antigen) is repeatedly provoking the immune response. If any latency-reactivation sites are revealed close to the involved joint and eradicated, it may soon be felt in lessening of the inflammation and swelling of the joint.
   In case of a painful hip joint, the relevant skin regions comprise those of the perineum, the genitalia and the groin and the perianal skin. In case of a painful knee, it is the skin of the knee and the shank.
   It is necessary to be patient as it may be that it will be progressively revealed that one's skin harbours hundreds of latency-reactivation sites and that some of them are quite large and deep. Such condition is most likely to occur in elderly persons since it appears that the causative agent takes many years to colonize the skin so extensively. In these instances it may be needed to carry on with the application of the invention to the skin for several years, perhaps with interruptions, as the roots of latency-reactivation sites appear very slowly as if coming up from the deeper tissue over many months.
   However, a relief from rheumatic pain, swelling and inflammation may be brought about by the application of the invention a long time before a complete eradication of the latency-reactivation sites from the skin is effected. A removal of even a part of necrosed latency-reactivation sites is followed by abatement in the magnitude of the immune response to the persistent antigen and, consequently, the progress of the disease is restrained.
   The eradication of the latency-reactivation sites arrests the development of the disease in the phase it has reached at that time, but injuries already caused to the joints and/or other organs by the inflammatory mechanisms may be irreversible. Nonetheless, a body freed from the latency-reactivation sites is a healthier one and thus more capable to repair at least some of the damage insofar as it is possible.
(d) In cases of subjects suffering from papulopustular skin diseases, e.g., acne, comedos and/or from psoriasis, tinea, onychia, paronychia, corns, callosities, alopecia, indolent tropical ulcers and/or other skin diseases, the application of the invention may best be directed first to the skin regions with clinical symptoms as latency-reactivation sites are likely to exist in their vicinity. The rest of the skin,which appears normal, may be treated later in the same manner as in cases of subjects with no clinical symptoms.

### Forms of industrial exploitation of the invention

(a) The manufacture of the product as claimed and its packaging, marketing and distribution to hospitals,sanatoria,physicians, veterinarians, wholesale and retail pharmaceutical and veterinary medicine outlets, other manufacturers and/or its in-house use in manufacturing the compositions and devices as claimed.
(b) The manufacture of the compositions and devices as claimed and their packaging, marketing and distribution to hospitals, sanatoria, physicians, veterinarians and wholesale and retail pharmaceutical and veterinary medicine outlets.

## Claims

1. Product for the prophylaxis, diagnosis and therapy of rheumatic, autoimmune, skin and connective tissue diseases of an unknown aetiology, characterized in that it consists of a salt, or salts, of iron (Fe) and a lipid, or lipids, that had all been heated together in a stirred vessel to the boiling point of the composition at the atmospheric pressure in the presence of water which then may have been completely or partly removed by distillation from the product which is to be used for topical administration onto the human or animal skin as a means of preventing or diagnosing and/or arresting the development of systemic lupus erythematosus, vasculitis, sarcoidosis, amyloidosis, rheumatoid arthritis, osteoarthrosis, rheumatic diseases, acne, comedos, papulopustular skin diseases, psoriasis, tinea, onychia, paronychia, corns, callosities, alopecia and indolent tropical ulcers.

2. Composition for topical application onto the human or animal skin comprising the product of Claim 1 in the form of an ointment, cream, paste, salve, gel, lotion, rinse, emulsion, aerosol spray, bath additive, wash or dispersion.

3. Device for topical application to the human or animal skin comprising a product according to Claim 1 or a composition according to Claim 2, such as a pad, a dressing, a poultice, a compress and/or a napkin.

4. Use of a product according to Claim 1 or a composition according to Claim 2 for obtaining a separate or combined prophylactic or diagnostic preparation and/or a medicine for topical application onto the human or animal skin.

5. Use of a product according to Claim 1 or of a composition according to Claim 2 or of a device according to Claim 3 for the manufacture of or for making a prophylactic, a diagnostic aid and/or a medicament for uses as claimed in Claim 1.

6. Use of a product according to Claim 1 or of a composition according to Claim 2 in in vitro cultivation of human, animal, plant or bacterial cells or viruses, or in the treatment of human or animal body tissues or fluids separated from the bodies they originate from and not to be returned to the same or other live bodies, or in research into the prophylaxis, diagnosis and/or therapy - not involving live humans or animals - of systemic lupus erythematosus, vasculitis, sarcoidosis, amyloidosis, rheumatoid arthritis, osteoarthrosis, rheumatic diseases, acne, comedos, papulopustular skin diseases, psoriasis, tinea, onychia, paronychia, corns, callosities, alopecia and indolent tropical ulcers.

7. Use of a product according to Claim 1 or of a composition according to Claim 2 for the manufacture of an ingredient or a culture medium or a culture medium additive for applications as mentioned in Claim 6.

## Patentansprüche

1. Mittel zur Vorbeugung, Diagnose und Therapie von rheumatischen, autoimmunen, Haut- und Bindegewebekrankheiten von unbekannter Aetiologie, dadurch gekennzeichnet, daß es aus einem Eisensalz, oder Eisensalzen, und einem Lipid, oder Lipiden,besteht, die alle in einem gerührten Gefäß zum Siedepunkt der Zusammensetzung unter dem atmosphärischen Druck zusammenerhizt worden waren, und dann das zugegenes Wasser könnte völlig oder teilweise durch Distillation vom Produkt entfernt worden sein, und dieses Produkt ist zur Anwendung bei der topischen Applikation auf die menschliche Haut als ein Mittel zur Vorbeugung oder zur Diagnose und/oder zum Stillstand der Entwicklung des systemischen Erythematodes, der Vasculitis, der Sarcoidose, der Amyloidose, des chronischen Gelenkrheumatismus, der Osteoarthritis, rheumatischer Krankheiten, der Akne, der Mitesser, papulopustularer Hautkrankheiten, der Schuppenflechte, der Fußmykose, der Nagelbettentzündung, der Nagelwallentzündung, der Hühneraugen, der Kallusse, der Alopezie und schmerzloser Tropengeschwüre.

2. Zusammensetzung zur topischen Applikation auf die menschliche oder tierische Haut enthaltend das Produkt gemäß Anspruch 1 in Form einer Salbe, Creme, Paste, Heilsalbe, Gel, Lotion, Spülung, Emulsion, Spray, Badezusatz, Washlotion oder Zerstreuung.

3. Vorrichtung zur topischen Applikation auf die menschliche oder tierische Haut, die das Produkt gemäß Anspruch 1 oder die Zusammensetzung gemäß Anspruch 2 enthält, solche als ein Bausch, ein Verband, ein Umschlag, eine Kompresse und/oder ein Tuch.

4. Verwendung eines Produktes gemäß Anspruch 1 oder einer Zusammensetzung gemäß Anspruch 2 zu seiner/ihrer Verarbeitung zu einem getrennten oder kombinierten vorbeugenden oder diagnostischen Präparate und/oder einem Medikamente zur topischen Applikation auf die menschliche oder tierische Haut.

5. Verwendung eines Produktes gemäß Anspruch 1 oder einer Zusammensetzung gemäß Anspruch 2 oder einer Vorrichtung gemäß Anspruch 3 zur Herstellung oder Zubereitung eines Vorbeugungsmittels, einer Diagnosenhilfe und/oder eines Medikamentes zu den Anwendungen gemäß Anspruch 1.

6. Verwendung eines Produktes gemäß Anspruch 1 oder einer Zusammensetzung gemäß Anspruch 2 zur *in vitro* Kultivierung von menschlichen, tierischen, pflanzlichen oder bakteriellen Zellen oder Viren, oder zur Behandlung von menschlichen oder tierischen Körpergeweben oder Flüssigkeiten, getrennt von Körpern aus denen sie stammen und nicht bestimmt in dieselbe Körper oder andere lebende Körper zurückgegeben zu werden, oder zur Forschung über die Vorbeugung, Diagnose und/oder Therapie - die nichts mit lebenden Menschen oder Tieren zu tun hat - des systemischen Erythematodes, der Vasculitis, der Sarcoidose, der Amyloidose, des chronischen Gelenkrheumatismus, der Osteoarthritis, rheumatisher Krankheiten, der Akne, der Mitesser, papulopustularer Hautkrankheiten, der Schuppenflechte, der Fußmykose, der Nagelbettentzündung, der Nagelwallentzündung, der Hühneraugen, der Kallusse, der Alopezie und schmerzloser Tropengeschwüre.

7. Verwendung eines Produktes gemäß Anspruch 1 oder einer Zusammensetzung gemäß Anspruch 2 zur Herstellung eines Bestandteiles oder eines Kulturmediums oder eines Kulturmediumzusatzes zu den Applikationen als sie sind genannt in Anspruch 6.

## Revendications

1. Produit pour la prophylaxie, le diagnostic et la thérapie des maladies rhumatismales, autoimmunes, de la peau et du tissu conjonctif d'étiologie inconnue, *caractérisé* en ce qu'il consiste en un sel, ou des sels, du fer (Fe) et en une lipide, ou des lipides, qui tous ensemble avaient été chauffés jusqu'au point d'ébullition de la composition sous la pression atmosphérique dans une cuve munie d'agitateur en présence de l'eau qui puisse ensuite être complétement ou partiellement éliminée par la distillation du produit qui est destiné à être employé en l'application topique sur la peau humaine ou animale comme un moyen de la prophylaxie ou du diagnostic et/ou pour arrêter le développement du systèmique lupus erythematosus, de la vasculite, de la sarcoïdose, de l'amyloïdose, du rhumatisme chronique polyarticulaire, de l'arthrose, des maladies rhumatismales, de l'acné, des comedones, des papulopustulaires maladies de la peau, du psoriasis, de la mycose, de l'onychie, du panaris, des cors au pied, des callosités, de la calvitie et des indolents ulcères tropicaux.

2. Composition pour l'application topique sur la peau humaine ou animale contenant le produit selon la revendication 1 sous forme de l'onguent, de la crème, de la pâte, du baume, de la gelée, de la lotion, de la rinçure, de l'émulsion, de la pulvérisation d'aérosol, de l'additif au bain, du lavage ou de la dispersion.

3. Dispositif pour l'application topique sur la peau humaine ou animale comprenant le produit selon la revendication 1 ou la composition selon la revendication 2, tel que le tampon, le pansement, le cataplasme, la compresse et/ou la serviette.

4. Utilisation du produit selon la revendication 1 ou de la composition selon la revendication 2 pour obtenir un moyen de prophylaxie ou de diagnostic ou un médicament séparé combiné pour l'application topique sur la peau humaine ou animale.

5. Utilisation du produit selon la revendication 1 ou de la composition selon la revendication 2 ou du dispositif selon la revendication 3 pour la fabrication ou la préparation d'un moyen de prophylaxie ou de diagnostic et/ou d'un médicament pour les emplois revendiqués dans la revendication 1.

6. Utilisation du produit selon la revendication 1 ou de la composition selon la revendication 2 pour la culture *in vitro* des cellules humaines, animales, végétales ou bactériennes ou pour celle du virus, ou pour le traitement des tissus ou des fluides de corps humain ou animal qui sont séparés des corps desquels ils proviennent et qui ne vont pas être retournés aux mêmes ou autres corps vivants, ou pour la recherche sur la prophylaxie, le diagnostic et/ou la thérapie - n'impliquant pas les humains ou les animaux vivants - du systémique lupus erythematosus, de la vasculite, de la sarcoïdose, de l'amyloïdose, du rhumatisme chronique polyarticulaire, de l'arthrose, des maladies rhumatismales, de l'acné, des comedones, des papulopustulaires maladies de la peau, du psoriasis, de la mycose, de l'onychie, du panaris, des cors au pied, des callosités, de la calvitie et des indolents ulcères tropicaux.

7. Utilisation du produit selon la revendication 1 ou de la composition selon la revendication 2 pour la fabrication d'un ingrédient ou d'un milieu de culture ou d'un additif au milieu de culture pour les applications mentionées dans la revendication 6.
